# EUROPÄISCHE PATENTSCHRIFT

(11) **EP 0 759 901 B1**
(45) Veröffentlichungstag und Bekanntmachung des Hinweises auf die Patenterteilung: **01.09.1999**
(21) Anmeldenummer: 95920008.0
(22) Anmeldetag: 09.05.1995
(51) Int. Cl.: C07C 227/18, C07C 229/12

(54) **VERFAHREN ZUR HERSTELLUNG N-Z-GESCHÜTZTER N-ALKYLIERTER AMINOSÄUREN**
PROCESS FOR PRODUCING N-Z PROTECTED N-ALKYLATED AMINO ACIDS
PROCEDE DE PREPARATION D'ACIDES AMINES N-ALKYLES A PROTECTION N-Z

(30) Priorität: 19.05.1994 DE 4417478
(43) Veröffentlichungstag der Anmeldung: 05.03.1997
(73) Patentinhaber: BASF AKTIENGESELLSCHAFT, 67056 Ludwigshafen (DE)
(72) Erfinder: KARL, Ulrich, D-67061 Ludwigshafen (DE); MÜLLER, Stefan, D-67346 Speyer (DE); DE POTZOLLI, Bernd, D-67098 Bad Dürkheim (DE)
(86) Internationale Anmeldenummer: EP9501752
(87) Internationale Veröffentlichungsnummer: WO9532180

(56) Entgegenhaltungen:
- WO-A-90/06914
- DE-A- 4 337 331

## Beschreibung

Die vorliegende Erfindung betrifft ein neues Verfahren zur Herstellung N-geschützter N-alkylierter Aminosäuren. N-alkylierte Aminosäuren stellen wichtige Bestandteile biologisch hochwirksamer Peptide dar. Beispiele für solche Peptide sind Cyclosporine (Wenger; Angew. Chem. 1985, 97, 88) und Dolastatine (G. Pettit; J. Nat. Prod. 1981, 44, 482).

Die Herstellung N-mono-alkylierter Aminosäuren ohne die Verwendung von N-Schutzgruppen hat keine praktische Bedeutung erlangt.

Die in der Literatur beschriebenen Verfahren erfordern teure und schwierig zu handhabende Reagenzien (Silberoxid, Natriumhydrid) im Überschuß (DE 2855786; Benoiton; Can. J. Chem 1977, 55, 906). Außerdem ist die oftmals gleichzeitig auftretende Veresterung der Säurefunktion (Olsen, J. Org. Chem. 1970, 35, 1912) unerwünscht und störend. Für den Einsatz in der Peptidchemie sind N-geschützte-N-alkylierte Aminosäurederivate besonders vorteilhaft, da die alkylierte Aminogruppe nicht reagieren kann und die Säurefunktion nicht freigesetzt werden muß.

Das bislang praktikabelste Verfahren wurde von Runge (WO 90/06914) vorgestellt, wobei t.Butyloxycarbonyl-geschützte Aminosäuren mit Alkyliodid versetzt werden und nach Zugabe von Kalium-tert.butanolat die Methylierung erfolgt.

Jedoch ist bei dem in WO 90/06914 beschriebenen Verfahren die gewählte Schutzgruppe für die Herstellung größerer Mengen N-geschützter-N-alkylierter Aminosäuren nicht optimal. Verwendet man statt der t.Butyloxycarbonyl- die Benzyloxycarbonyl-Schutzgruppe (im folgenden mit Z abgekürzt) unter den Bedingungen nach WO 90/06914, so erhält man die gewünschte N-alkylierte Aminosäure nur in sehr mäßiger Ausbeute nebst vielen Nebenprodukten.

Die Verwendung der Z-Schutzgruppe unter den von Runge beschriebenen Bedingungen ist bislang nicht beschrieben worden, vermutlich deshalb, weil sich aus der einschlägigen Literatur (Fieser & Fieser; Reagents for Organic Synthesis Vol. 1, 110 Wiley 1967; Houben-Weyl Bd. XV/1, 64) ergibt, daß die Z-Schutzgruppe unter alkalischen Bedingungen leicht hydrolysiert.

Überraschend wurde gefunden, daß bei geeigneter Reaktionsführung das gewünschte Produkt in sehr guter Ausbeute und Reinheit erhalten werden kann.

Die vorliegende Erfindung betrifft ein Verfahren zur Herstellung N-geschützter-N-alkylierter Aminosäuren der Formel I: 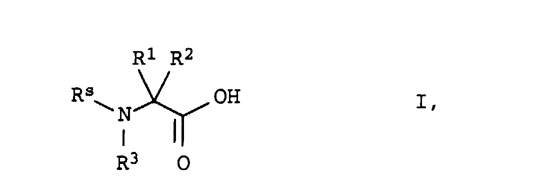 worin
- R^{S}: für einen gegebenenfalls beliebig substituierten Benzyloxycarbonylrest,
- R¹: für die Seitenkette einer proteinogenen Aminosäuren oder deren Derivat,
- R²: für Wasserstoff, Alkyl, Alkenyl, Alkinyl, Aryl oder Heteroaryl und
- R³: für C₁₋₂-Alkyl
stehen, dadurch gekennzeichnet, daß man eine Verbindung der Formel II: 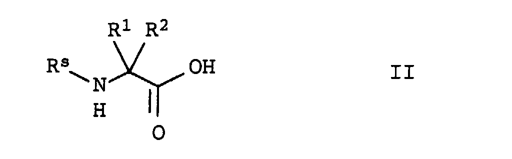 worin R¹, R² und R^{S} die oben angegebene Bedeutung haben, mit einer Lösung von Natrium- oder Kalium-tert.butanolat in einem nicht-protischen organischen Lösungsmittel versetzt und anschließend ein C₁₋₂-Alkylhalogenid zusetzt.

In Formel I haben die Substituenten vorzugsweise folgende Bedeutung:
- R¹: der Rest einer proteinogenen Aminosäuren, insbesondere H, CH₃, CH₂-CH₂-CH₃, CH(CH₃) -CH₂-CH₃, CH₂-CH- (CH₃)₂, CH₂-C₆H₅, CH₂-C₆H₄O-C(CH₃)₃, CH₂-C₆H₄OCH₂-C₆H₅, CH₂-O-CH₃, CH₂-O-C(CH₃)₃, CH₂-O-Si(CH₃)₃, CH₂-O-CH₂-C₆H₅, CH₂-C₆H₄O-Si(CH₃)₃, CH(CH₃)-O-CH₃, CH(CH₃)-O-C(CH₃)₃, CH(CH₃)-O-Si(CH₃)₃, CH(CH₃)-O-CH₂-C₆H₅, CH₂-S-C (C₆H₅)₃, CH₂-S-CH(C₆H₅)₂, CH₂-S-CH₂-C₆H₅, CH₂CH₂CH₂CH₂N- (CO)₂C₆H₄, besonders CH-(CH₃)₂,
- R²: H, C₁₋₄-Alkyl, insbesondere CH₃, CH₂-CH₃, CH₂-CH₂-CH₃, CH(CH₃)-CH₂-CH₃, CH₂-CH₂-CH₂-CH₃, CH₂-CH-(CH₃)₂, C(CH₃)₃, CH₂-CH=CH₂, CH₂-C≡CH, C₆H₅, C₆H₄CH₃,
- R³: Methyl,
- R^{S}: COOCH₂-C₆H₅ (=Z), COOC(CH₃)₂-C₆H₅ (=DMZ), COOCH₂-C₆H₄(4-Br) (=BZ), COOCH₂-C₆H₄(4-Cl) (=CZ), COOCH₂-C₆H₄(3-Cl) (=3CZ), COOCH₂-C₆H₄(2-Cl) (=2CZ), COOCH₂-C₆H₄(4-OCH₃) (=MOZ), COOCH₂-C₆H₄(4-NO₂) (=NZ), COOCH₂-C₆H₄(2-NO₂) (=2NZ), COOCH₂-C₆H₄(4-OCOCH₃) (=AcOZ).

Besonders bevorzugt als Schutzgruppe ist Z.

Die erfindungsgemäße Reaktion wird zweckmäßig unter einem inerten Schutzgas wie Helium oder Argon durchgeführt. Besonders vorteilhaft ist der Einsatz von Stickstoff.

Als Lösungsmittel für die Reaktion sind nicht-protische Lösungsmittel geeignet, insbesondere Tetrahydrofuran, 1,2-Dimethoxyethan, Diethoxymethan, Dioxan, Dichlormethan, Trichlormethan, Tetrachlormethan sowie N,N-Dimethylethylenharnstoff, N,N-Dimethylpropylenharnstoff und N-Methylpyrrolidon.

Vorzugsweise findet Tetrahydrofuran Verwendung.

Als Basen werden Natrium-t.-butanolat oder Kalium-t.-butanolat verwendet. Vorzugsweise wird Kalium-t.-butanolat eingesetzt. Es werden 2,2 bis 10, vorzugsweise 3,5 bis 6 Äquivalente der Base bezogen auf II verwendet.

Als Alkylierungsmittel sind Methyl- und Ethylbromid und insbesondere Methyl- und Ethyliodid geeignet. Es werden 1,2 bis 6, insbesondere 2,5 Äquivalente, des Alkylierungsmittels bezogen auf II verwendet.

Die Reaktion gelingt bei Temperaturen zwischen -40°C und +100°C. Vorteilhaft wird sie bei Temperaturen zwischen -10°C und +20°C durchgeführt.

Die Aufarbeitung der Reaktion kann durch Destillation, Extraktion, Kristallisation, Chromatographie oder eine Kombination derselben erfolgen. Bevorzugt wird eine Extraktion nach Ansäuern durchgeführt und anschließend die Verbindung I auskristallisiert.

Zum Ansäuern können Säuren wie Salzsäure, Schwefelsäure, Phosphorsäure, Bromwasserstoffsäure, Essigsäure, Methansulfonsäure verwendet werden. Vorzugsweise wird Schwefelsäure verwendet. Als Extraktionsmittel können mit Wasser nicht mischbare Lösungsmittel wie Pentan, Hexan, Heptan, Octan, Petrolether, Ethylacetat, Diethylether, Di-Isopropylether, Methyl-t.Butylether, Dichlormethan, Chloroform, Tetrachlorkohlenstoff, Benzol, Toluol, Xylol verwendet werden, besonders bevorzugt ist Toluol. Die Kristallisation kann aus organischen Lösungsmitteln wie Pentan, Hexan, Heptan, Octan, Petrolether, Ethylacetat, Dichlormethan, Chloroform, Tetrachlorkohlenstoff, Benzol, Toluol, Xylol, Aceton, Butan-2-on, Methanol, Ethanol, n-Propanol, iso-Propanol, Diethylether, Di-Isopropylether, Methyl-t.Butylether sowie Gemischen derselben erfolgen, bevorzugt werden Toluol sowie Toluol/Heptan-Gemische verwendet. Enthalten die Aminosäuren weitere reaktive Gruppen, wie beispielsweise Cystein, Serin, Tyrosin, Lysin, Threonin, so müssen diese während der Umsetzung geschützt sein.

Besondere Vorteile des Verfahrens liegen darin, daß
1) kostengünstige Einsatzstoffe mit vorteilhaften Handhabungseigenschaften verwendet werden (Z-Aminosäuren),
2) keine gleichzeitige Veresterung stattfindet,
3) die Ausbeute gegenüber literaturbekannten Verfahren stark ansteigt,
4) ein Produkt hoher Reinheit isoliert wird, welches ohne weitere Reinigung für Folgereaktionen verwendet werden kann und
5) keine Racemisierung erfolgt.

### Beispiel 1

### (S)-Z-N-Methyl-Isoleucin

Zu einer Lösung von 13,25 g (S)-Z-Isoleucin und 17,75 g Methyliodid in 70 ml THF wurden bei -10-0°C 19,6 g Kalium-tert.-butanolat gelöst in 100 ml THF im Verlauf 1 h zugegeben. Danach wurden 18,9 g Methyliodid so zugegeben, daß die Temperatur zwischen 0 und 5°C blieb. Anschließend wurde 2 h bei -10°C und danach 2 h bei 20°C gerührt. Durch Zugabe von 200 ml Wasser wurde die Reaktion abgebrochen. Die organische Phase wurde abgetrennt und die wäßrige Phase mit 100 ml Toluol extrahiert. Die vereinigten organischen Phasen wurden verworfen. Die wäßrige Phase wurde angesäuert und mit Methylenchlorid extrahiert. Die Methylenchlorid-Phase wurde zur Trockene eingeengt. Das so erhaltene Rohprodukt wurde aus Toluol/Heptan umkristallisiert. Es wurden 7,5 g Produkt mit einer Reinheit von > 96 % isoliert.
Fp.: 50,5-54,3°C

Analog Beispiel 1 wurden hergestellt:
2. (S)-Z-N-Methyl-Valin
   Ausbeute: 85 %; Reinheit: 99 % (HPLC)
   Fp.: 69,6-71,2°C
   [α]_{D} = -85,0°C (c=0,99 in CH₃OH)
3. (S)-Z-N-Methyl-Valin
   (statt Methyliodid wurde Methylbromid verwendet)
   Ausbeute: 73,5 %; Reinheit 98 % (HPLC)
4. (S)-Z-N-Methyl-Phenylalanin
   Ausbeute: 81 %; Reinheit 97,4 % (HPLC)
   Fp.: 66,5-68,0°C
   [α]_{D} = - 65,1°C (c=1,0 in CH₂Cl₂)
5. (S)-Z-N-Methyl-Serin (tbu)
   Ausbeute: > 95 %; Reinheit 96 % (HPLC)
   ¹H-NMR [270MHz, CDCl₃] : 1.2 (9H), 3.0 (3H),3.7-3.9 (2H), 4.65-4.8 (2H), 5.2 (2H), 7.3 (5H), 9.8 [1H]

Analog lassen sich folgende Verbindungen herstellen:
(S)-Z-N-Methyl-Alanin; (R)-Z-N-Methyl-Alanin; (+/-)-Z-N-Methyl-Alanin;
(S)-Z-N-Methyl-Leucin; (R)-Z-N-Methyl-Leucin; (+/-)-Z-N-Methyl-Leucin;
(S)-Z-N-Methyl-Threonin(O-Benzyl); (R)-Z-N-Methyl-Threonin(O-Benzyl); (+/-)-Z-N-Methyl-Threonin(O-Benzyl);
Z-N-Methyl-Glycin;
(S)-Z-N-Methyl-Methionin; (R)-Z-N-Methyl-Methionin; (+/-)-Z-N-Methyl-Methionin;
(S)-Z-N-Methyl-Cystein(S-Benzyl); (R)-Z-N-Methyl-Cystein(S-Benzyl); (+/-)-Z-N-Methyl-Cystein(S-Benzyl);
Z-N-Ethyl-Glycin.

## Patentansprüche

1. Verfahren zur Herstellung N-geschützter N-alkylierter Aminosäuren der Formel I: 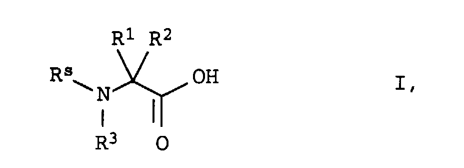 worin
R^{S} für einen gegebenenfalls beliebig substituierten Benzyloxycarbonylrest,
R¹ für die Seitenkette einer proteinogenen Aminosäure oder deren Derivat,
R² für Wasserstoff, Alkyl, Alkenyl, Alkinyl, Aryl oder Heteroaryl und
R³ für C₁₋₂-Alkyl
stehen, dadurch gekennzeichnet, daß man eine Verbindung der Formel II: 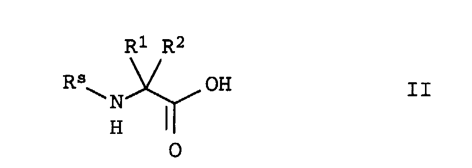 wobei R¹, R² und R^{S} die oben angegebene Bedeutung haben, mit einer Lösung von Natrium- oder Kalium-tert.-butanolat in einem nicht-protischen organischen Lösungsmittel versetzt und anschließend ein C₁₋₂-Alkylhalogenid zusetzt.

2. Verfahren nach Anspruch 1, dadurch gekennzeichnet, daß als Base Kalium-tert.butanolat und als Alkylierungsmittel Methyliodid oder -bromid verwendet wird.

3. Verfahren nach Anspruch 1 und 2, dadurch gekennzeichnet, daß Rs für den Benzyloxycarbonylrest steht.

## Claims

1. A process for preparing N-protected N-alkylated amino acids of the formula I: 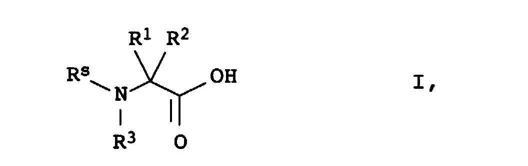 where
R^{S} is a benzyloxycarbonyl radical optionally substituted in any way,
R¹ is the side chain of a proteinogenous amino acid or derivative thereof,
R² is hydrogen, alkyl, alkenyl, alkynyl, aryl or hetaryl and
R³ is C₁₋₂-alkyl
which comprises mixing a compound of the formula II: 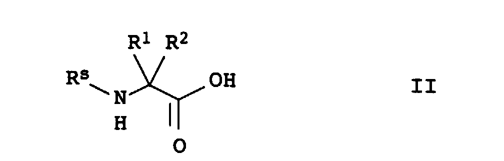 where R¹, R² and R^{S} have the abovementioned meanings, with a solution of sodium or potassium tert-butanolate in a non-protic organic solvent and subsequently adding a C₁₋₂-alkyl halide.

2. A process as claimed in claim 1, wherein potassium tert-butanolate is used as base, and methyl iodide or bromide is used as alkylating agent.

3. A process as claimed in claim 1 or 2, wherein R^{s} is the benzyloxycarbonyl radical.

## Revendications

1. Procédé pour la préparation d'aminoacides alkylés à l'azote et protégés à l'azote, qui répondent à la formule I 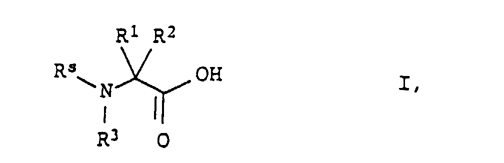 dans laquelle
R^{S} représente un groupe benzyloxycarbonyle portant éventuellement un substituant quelconque,
R¹ représente la chaîne latérale d'un aminoacide protéinogène ou d'un de ses dérivés,
R² représente l'hydrogène, un groupe alkyle, alcényle, alcynyle, aryle ou hétéroaryle et
R³ représente un groupe alkyle en C1-C2,
caractérisé par le fait que, à un composé de formule II 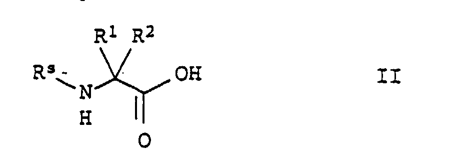 dans laquelle R¹, R² et R^{S} ont les significations indiquées ci-dessus,
on ajoute une solution de tert-butylate de sodium ou de potassium dans un solvant organique non protonique puis on ajoute un halogénure d'alkyle en C1-C2.

2. Procédé selon la revendication 1, caractérisé par le fait que l'on utilise, en tant que base, le tert-butylate de potassium et, en tant qu'agent alkylant, l'iodure ou le bromure de méthyle.

3. Procédé selon les revendications 1 et 2, caractérisé par le fait que R^{S} représente le groupe benzyloxycarbonyle.
